# EUROPEAN PATENT APPLICATION

(11) **EP 0 754 450 A1**
(43) Date of publication of application: **22.01.1997**
(21) Application number: 96202765.2
(22) Date of filing: 16.10.1990
(51) Int. Cl.: A61K 9/00, A61K 31/315, A61K 31/375

(54) **Vitamin-mineral treatment methods and compositions**

(62) Divisional of application: 90916875.9
(71) Applicant: MAYOR PHARMACEUTICALS LABORATORIES,INC., Phoenix, AZ 85034 (US)
(72) Inventor: Deihl, Joseph A., Phoenix, Arizona 85034 (US)
(74) Representative: MacGregor, Gordon

(57) **Abstract**

A pharmacological treatment method in which a spray composition is injected into the oral cavity in a multiplicity of aliquot doses during waking hours. The composition includes a soluble vitamin-mineral, a soluble flavouring agent and a non-toxic solvent for these compounds. In preferred embodiments the composition may include a non-toxic zinc compound, a combination of non-toxic zinc compound and Vitamin C, Vitamin A, or Vitamin B₁₂.

## Description

This invention relates to the manufacture of a pharmacological composition for treatment of the common cold.

US Patent 4,525,341 discloses methods for administering vitamins to humans by spraying a liquid vitamin composition into the mouth. The vitamin(s) were present in these compositions in a diet supplemental amount. The sprayable compositions contained a breath freshener and, optionally, a flavouring agent. The liquid carrier was a non-toxic alcohol, or an aqueous alcohol or water.

Urbach described the clinical use of Vitamin A buccal tablets in the treatment of leukoplakia and lichen planus in Bull. Ass. Milit. Derm 6: 17, 1957.

The local therapy of oral leukoplakia with Vitamin A troches was described by Mulay and Urbach in AMA Arch. Dem. and Syph. 78: 637-58, 1958.

Eby, Davies and Halcomb reported that zinc gluconate lozenges, dissolved in the mouth every two wakeful hours after an initial double dose, shortened the average duration of common colds by about seven days. Problems with palatability, taste distortion, mouth irritation, emetic properties and nausea were reported. Antimicrobial Agents and Chemotherapy, Vol. 25, No.1, Jan. 1985, p. 20-24. (See also US-A-4956385).

The intra-nasal introduction of diverse drugs has been suggested and tested, including Vitamin B12, metoclopramide (cancer chemotherapeutant), insulin, LHRH hormone (contraceptive), lidocaine (headache medicine), alpha-interferon and enviroxime (rhinoviricides) and measle and flu vaccines. Some of these treatments induced side effects such as irritation and nasal bleeding.

JP-A-63096123 discloses the administration, including spraying, of a vitamin A zinc salt for anti-inflammatory purposes.

US-A-4826683 discloses the administration by nasal spray of a mixture including vitamin C and a zinc compound as a decongestant.

Thus, the prior art discloses that vitamins and other medicinal compositions can be administered by mucosal absorption from a composition sprayed in to the oral cavity and that nasal administration of certain similar compositions may be feasible. However, the prior art fails to teach or fairly suggest the use of certain specific combinations of vitamins/minerals, administered in a specific, controlled fashion, to achieve particular medical and/or biochemical effects without adverse side effects. In particular, the prior art does not disclose the pharmacologic effect of topical application of certain vitamin, mineral or vitamin-mineral oral spray compositions in the treatment of oral lesions. Further, the prior art fails to disclose specific-administration regimens for vitamin sprays to achieve specific pharmacologic and nutritional results.

Accordingly, the principal object of the present invention is to provide improved methods and compositions for administering vitamins, vitamin-mineral and mineral compositions in to the human body to achieve specific pharmacologic and/or nutritional results.

Another object of the invention is to provide such methods and compositions which can be self-practiced and administered by human patients with only limited instruction and minimal supervision by medically trained personnel.

A further object of the invention is to provide methods and compositions which may be conveniently employed to administer vitamins, vitamin-mineral and mineral compositions with minimal side effects.

These and other, further and more specific objects and advantages of the invention will be apparent from the following description, taken in conjunction with the working examples, which are presented as illustrations of the presently preferred practice of the invention and which are not intended as limitations on the scope thereof.

### BRIEF SUMMARY OF THE INVENTION

Briefly, in accordance with the present invention, I provide improvements in the prior art methods disclosed in my above-identified issued U.S. patent. The method which I previously disclosed includes the steps of forming an air spray of a composition comprising a water-soluble vitamin, and a water soluble flavoring agent in an aqueous carrier, spraying said composition into the oral cavity and repeating the spraying step at spaced periods throughout the waking hours. According to the present invention this method is improved by establishing a desired total dose for administration during a daily dosage period, selecting a predetermined concentration of a water soluble vitamins and/or minerals which will provide an aliquot dosage and repeating administration of such aliquot dose during the daily dosage period, to deliver the desired total dose.

In one presently preferred embodiment of the invention the spray composition includes a non-toxic water soluble zinc compound.

According to another embodiment of the invention the spray composition includes Vitamin A.

In another preferred embodiment the composition includes Vitamin B12.

According to still another preferred embodiment of the invention, oral lesions are treated by topical application of a Vitamin A spray.

According to a still further and preferred embodiment of the invention, common colds are treated by oral administration of a spray composition comprising a solution of a non-toxic zinc compound.

### DETAILED DESCRIPTION OF THE INVENTION

The invention will now be described with such particularity as to enable any person skilled in the pertinent art to practice the invention without extensive experimentation.

In the administration of biologically active agents, including drugs, medicines and other compositions which are administered for pharmacological effect, it is normally required that a minimum concentration of the agent be established in the body, e.g., in the tissue or blood stream, and that such minimum concentration be maintained for a predetermined time period, as required to obtain the desired effect. Typically, these requirements are determined by empirical tests and the test results translated to a dosage and a pattern of repetition of such dosages as required to maintain the desired minimum body concentration of the agent. According to the prior art, such timed aliquot dosages have been administered orally, intravenously, intradermally or by inhalation to ultimately provide the desired total dosage. However, according to my present knowledge, the regimen of timed aliquot doses, substantially spaced throughout the wakeful period has not been practiced via mucosal absorption of the agent from solutions thereof sprayed into the oral cavity.

According to the broadest aspects of my invention, desired total dosages of specific soluble vitamin compositions are predetermined by appropriate clinical studies in which the compositions are sprayed into the oral cavity. Similarly, suitable aliquot dosages and administration intervals are predetermined based on observed clinical effects, after the spray administration of such compositions, e.g. by blood and/or tissue analysis and similar techniques. Having so predetermined the concentration of the vitamin solution and the spray dosage and interval, the method can be practiced by persons having only limited medical training and even by the patients themselves.

To improve the palatability of such spray compositions I preferably include suitable flavoring agents in the spray composition, which may include any suitable flavoring agents which are soluble in the spray compositions. For example, flavoring agents such as sucrose, fruit juices, e.g. orange, apple, grape and pineapple juices and concentrates, are effectively employed.

The liquid carrier for the vitamin and/or mineral agents in the spray is chosen with reference to chemical compatibility with the active agents, including the ability to dissolve such agents, and non-toxicity as well as secondary factors such as taste, volatility, etc. The solvents for the active vitamin and/or minerals can include, for example, water, aqueous alcohols or 200-proof spirits.

The spray can be formed by any suitable technique, including conventional air pump atomizers and conventional compressed gas atomizers. The specific type of dispenser is not highly critical and will be chosen with reference to the specific desired effects, such as whether the spray is intended for a general application to the buccal mucosa for optimum absorption of the active agents into the blood stream or for local application to specific sites in the oral cavity to achieve topical effects. The selection of specific atomizer apparatus can be made by routine experimentation by persons skilled in the art, having regard for this disclosure.

Specific pharmacological effects can be obtained in accordance with my invention include acceleration of healing of oral lesions by topical applications of retinoids, the biologically active form of Vitamin A. Additional specific biological effects of my spray retinoid compositions include the treatment of night blindness, immunostimulation, and free radical scavenging.

Retinyl propionate, dissolved in the spray is hydrolyzed to retinyl after oral mucosal absorption. Retinol is then transported into blood plasm, bound to a alpha one-globulin which protects it from metabolism and delivers it to specific sites on the target cell surface. Another protein carrier then aids in the penetration of he vitamin in the cell where it is biological activity takes place.

The biological activity of the retinoids are diverse. For example, in the epithelium Vitamin A plays a major role in the induction and differentiation of mucus secreting and keratinizing tissue. Immunostimulation effects are obtained by therapeutic levels of Vitamin A which increase circulating T-cells.

Another specific biological effect which may be attained according to my invention is the reduction in severity of common colds by intraoral application of non-toxic zinc solutions which exhibit rhinoviricidal activity. The inclusion of Vitamin C in such compositions improves the effectiveness of such treatment.

Other specific vitamin and/or mineral compositions which can be advantageous employed in the practice of may invention will readily occur to those skilled in the art, having regard for this disclosure.

### Working Examples

The following examples will serve to illustrate the practice of my invention to those skilled in the art and to identify the presently preferred embodiments and best modes of practice of my invention. These working examples are intended as illustrations of the practice of the invention and not as limitations of the scope thereof.

### EXAMPLE I

This example illustrates the preparation of a sprayable Vitamin A composition. Table 1 identifies the final compositions by ingredient and weight percentage.

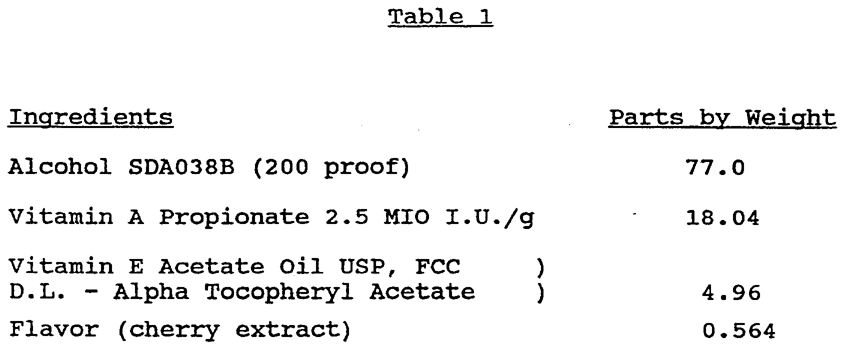

The Vitamin A is slowly added to the pre-measured alcohol and completely dissolved. The tocopheryl is then slowly added until completely dissolved and the resultant solution is gently agitated for 15-20 minutes after which the flavoring is added and uniformly dissolved in the final mixture.

### EXAMPLE II

This example illustrates the preparation of a suitable sprayable zinc-Vitamin C composition. Table II identifies the final compositions by ingredient and weight percentage.

**Table 2**

| Ingredients | Parts by Weight |
|---|---|
| Pharmaceutical grade water (carrier) | 54.53 |
| Ascorbic Acid | 1.64 |
| Sodium Bicarbonate | 0.14 |
| Glycerine | 6.8 |
| Potassium Sorbate | 0.0765 |
| EDTA | 0.0082 |
| Zinc Gluconate | 1.09 |
| L-Lysine | 0.55 |
| Glycine | 0.55 |
| Fruit Juice (Flavoring) | 0.27 |
| Sucrose (Flavoring) | 32.79 |
| Magnasweet (GA-110) (Flavor Enhancer) | 0.27 |
| Emulsifier (Tween-80) | 0.55 |
| Trace Bioflavonoids (Rutin, Hesperidan, 27 others) | 0.27 |
| Orange Flavoring | 0.068 |
| Peppermint Oil | 0.492 |

The water at 85-92°F is measured into a sterile stainless steel vat. The glycerine and potassium sorbate is added to the water. The ascorbic acid and bicarbonate are separately mixed in a portion of the solution from the vat to stabilize the ascorbic acid and this solution is then added to the main batch in the vat. The following ingredients are then added singly with stirring to obtain complete solution thereof before the next ingredient is added: EDTA, zinc gluconate, L-lysine/glycerine, fruit juice/sucrose, flavor enhancer.

In a separate stainless steel container, the bioflavonoids are added to the emulsifier with constant mixing. Peppermint oil and then the orange flavoring are added to the container and mixed. The contents of the separate container are then slowly added to the vat to form a creamy light viscous solution.

### EXAMPLE III

This example illustrates the preparation of a sprayable Vitamin B12 composition. Table 3 identifies the final composition by ingredient and weight percentage.

| Ingredients | Parts by Weight |
|---|---|
| Pharmaceutical Grade Water (Carrier) | 66.43 |
| Sucrose (Flavoring Agent) | 24.03 |
| Sorbistat K (Preservative) | 0.091 |
| Glycerine | 8.12 |
| Orange Juice (Flavoring Agent) | 0.325 |
| Emulsifier (Tween 80) | 0.49 |
| Peppermint Oil | 0.519 |
| Cyanocobalamin (99.5%) | 0.01169 |

The water at 86-92°F is measured into a sterile stainless steel vat. The sorbistat K, glycerine, sucrose and fruit juice are then slowly added and completely dissolved before the next ingredient is added. The cyanocobalamin is then added slowly and completely dissolved, leaving a clear deep red solution after approximately 30 minutes. Meanwhile, the emulsifier and peppermint oil are mixed to a subtle cloudy mixture, which requires approximately 20 minutes. Then this cloudy mixture is slowly added to the contents of the vat with final mixing.

### EXAMPLE IV

This example illustrates the use of a Vitamin A spray of Example I in the treatment of oral lesions.

### Procedure

Forty-nine adult patients with oral lesions were treated for a period of four weeks with a topical spray of the Vitamin A-alcohol formulation of Example I. The spray was contained in an aerosol container five inches in length and two inches in circumference. Females of child bearing age were asked to take appropriate birth control measures while on the study and for two week after stopping the medication.

Patients were instructed to spray the lesions eight times daily. The total dosage of Vitamin A delivered to the lesions in this fashion was 200,000 u/day.

Table I illustrates the number of patients with each disease treated.

**TABLE I**

| Number of patients with oral lesions. | |
|---|---|
| Disease | Number |
| Pemphigus | 4 |
| Leukoplakia | 12 |
| Glossitis | 3 |
| Geographic Tongue | 6 |
| Lichen Planus | 14 |
| Aphthous Stomatitis | 10 |

Six patients did not return for follow-up.

The status of the disease and the response to treatment was evaluated by the same investigator weekly for a period of four weeks.

It was emphasized to the patients with leukoplakia that the condition might be precancerous and that other modalities of therapy were available.

A double blind procedure was not used since the effect of topical Vitamin A in oral lesions has been known for many years and only the effect of the application and delivery system was of relevance.

### Results

Forty-three of the forty-nine patients completed the four week course of treatment. Table II illustrates the percentage of improvement in the patients that completed the four week course of treatment.

**Table II**

| Percentage of improvement at end of four week period in each disease category. | | |
|---|---|---|
| Disease | Number of Patients | Percent Improved |
| Pemphigus | 3 | 60 |
| Leukoplakia | 10 | 50 |
| Glossitis | 2 | 80 |
| Geographic Tongue | 6 | 95 |
| Lichen Planus | 12 | 65 |
| Aphthous Stomatitis | 10 | 75 |

### EXAMPLE V

The procedure of Example II is repeated except that ascorbic acid is omitted from the composition.

### EXAMPLE VI

The procedure of Example II is repeated except that zinc gluconate is omitted from the composition.

### EXAMPLE VII

The procedure of Example II is repeated except that both ascorbic acid and zinc gluconate are omitted from the composition.

### EXAMPLE VIII

The compositions of Examples II, V, VI and VII are administered by spraying every two hours during wakeful periods into the mouths of patients having the symptoms of common colds. The total daily doses administered are approximately 300 mg. zinc gluconate in the compositions of Examples II and V.

After seven days of these treatments the results are:

| Spray Compositions | % Patients Asymptomatic |
|---|---|
| Ex. II | 85 |
| Ex. V | 75 |
| Ex. VI | 60 |
| Ex. VII | 45 |

## Claims

1. A method for the preparation of a pharmaceutical or nutritional composition including a vitamin and/or mineral agent, the method comprising the steps of establishing a desired total dose for administration during a daily dosage period, selecting a concentration of a soluble vitamin and/or mineral which will provide the desired total dose when administered in aerosol form as a series of aliquot doses at predetermined spaced intervals during waking hours into the oral cavity, and charging the composition to a spray dispenser.

2. A method as claimed in Claim 1 wherein the step of selecting a concentration of a soluble vitamin and/or mineral further includes the step of determining the concentration by empirical tests and translating the tests to the aliquot dosage and a pattern of repetition of such dosages to maintain the desired minimum body concentration of the vitamin and/or mineral.

3. A method for the preparation of a pharmaceutical or nutritional composition including a vitamin and/or a mineral agent for administration in aerosol form to the oral cavity, the method comprising charging the composition to a spray dispenser, characterised by establishing a desired total dose of the vitamin and/or mineral for administration in aerosol form to the oral cavity during a daily dosage period, determining a concentration of a soluble vitamin and/or mineral which will provide the desired total dose when administered in aerosol form as a series of aliquot doses at predetermined spaced intervals during waking hours into the oral cavity, and producing a pharmaceutical or nutritional composition including the predetermined vitamin and/or mineral concentration.

4. The method according to Claim 3, in which the mineral agent comprises a non-toxic water soluble zinc compound.

5. The method according to Claim 3 or Claim 4, in which the vitamin is Vitamin C.

6. A composition prepared according to the method of any preceding claim, in combination with instructions to spray a series of aliquot doses at predetermined spaced intervals during waking hours into the oral cavity of a patient.
